Europäisches Patentamt

European Patent Office

Office européen des brevets

(11) Numéro de publication: **0 225 832**
A2

## (12) DEMANDE DE BREVET EUROPEEN

(21) Numéro de dépôt: 86402682.8

(22) Date de dépôt: 03.12.86

(51) Int. Cl.4: **A 61 K 35/78**
A 61 K 37/22

(30) Priorité: 06.12.85 FR 8518086

(43) Date de publication de la demande:
16.06.87 Bulletin 87/25

(84) Etats contractants désignés:
AT BE CH DE ES GB GR IT LI LU NL SE

(71) Demandeur: Desjonqueres, Stéphane
7 rue du Chant des Oiseaux
F-78360 Montesson (Les Yvelines) (FR)

(72) Inventeur: Desjonqueres, Stéphane
7 rue du Chant des Oiseaux
F-78360 Montesson (Les Yvelines) (FR)

(74) Mandataire: Herrburger, Pierre
Cabinet Pierre Herrburger 115, boulevard Haussmann
F-75008 Paris (FR)

(54) Composition médicamenteuse destinée à être utilisée pour le traitement des hémorroides.

(57)   a) Composition médicamenteuse destinée à être utilisée pour le traitement des hémorroïdes ;
   b) caractérisé en ce qu'elle contient au moins une huile naturelle d'origine végétale hyperoxygénée par saturation en oxygène et exposition intensive et contrôlée aux ultraviolets de façon à présenter un taux de péroxydation compris entre 30 et 300 exprimé en milli-équivalents et une teneur en glycérides oxydés comprise entre 5 et 40, la définition de cette huile en spectrophotométrie UV étant de 1 à 6 pour le facteur E 270 et de 8 à 60 pour le facteur E 232.

EP 0 225 832 A2

**Description**

" Composition médicamenteuse destinée à être utilisée pour le traitement des hémorroïdes "

La présente invention se rapporte à une composition médicamenteuse destinée à être utilisée pour le traitement des hémorroïdes.

La poussée hémorroïdaire comporte une trilogie traditionnelle: douleur, inflammation, saignement. Les patients souffrant d'hémorroïdes savent, malheureusement, à quel point celles-ci peuvent être gênantes dans la vie quotidienne et souhaitent ardemment qu'on leur propose un traitement susceptible de mettre fin à leurs maux.

Malgré de nombreuses recherches, on n'a, cependant, par encore pu trouver de médication anti-hémorroïdaire susceptible de donner entière satisfaction, c'est-à-dire d'une bonne efficacité. d'une utilisation simple et en même temps dépourvue d'effets secondaires ; pour cette raison, de nombreux patients se tournent vers la chirurgie pour tenter de trouver un soulagement.

Indépendamment de cette solution extrême qui n'est par toujours satisfaisante, le traitement antihémorroïdaire le plus souvent proposé consiste en l'application locale d'une pommage à base d'extrait hydroalcoolique de levuer de bière, d'huile de foie de flétan, de nitrate de phényl mercure et d'essence de thym commercialisée par les Laboratoires WHITEHALL de Clichy sous la dénomination "PREPARATION H".

Cette composition se montre malheureusement souvent d'une efficacité insuffisante et son utilisation n'est pas toujours dépourvue d'effets secondaires.

La présente invention a pour objet de remédier à ces inconvénients en proposant une composition médicamenteuse destinée à être utilisée pour le traitement des hémorroïdes qui soit susceptible de donner entière satisfaction.

A cet effet, on s'est aperçu, de manière surprenante, que certaines huiles naturelles hyperoxègénées, possédaient des qualités propres leur permettant de se montrer nettement supérieures aux médicaments analogues utilisés jusqu'à présent pour le traitement des hémorroïdes, qu'il s'agisse de produits d'automédication (PREPARATION H) ou de produits de prescription (cortisoniques, anesthésiants de contact).

La possilbité d'utiliser des péroxydes de corps gras pour le traitement de certaines affections a été mentionnée pour la première fois dans le brevet français n° 2 330 M ; l'utilisation des compositions décrites dans ce document n'a, malheureusement, pas permis d'aboutir aux résultats espérés, notamment, à cause de problèmes de tolérances, et pour cette raison, les recherches visant à améliorer ces compositions ont été interrompues pendant un certain nombre d'années.

Ces recherches ont été reprises récemment, en liaison avec l'intérêt accru du prublic pour les produits naturels, et en particulier, pour la médecine dite "douce".

On a, ainsi, pu, selon l'invention, mettre en évidence des qualités pharmacologiques des huiles hyperoxygénées préparées, par exemple, conformément à la mise en oeuvre du procédé décrit dans le brevet français n° 2 461 744.

L'invention concerne une composition médicamenteuse renfermant de tels corps gras.

Cette composition est caractérisée en ce qu'elle contient au moins une huile naturelle d'origine végétale hyperoxégénée par saturation en oxygène et exposition intensive et contrôlée aux ultraviolets, de façon à présenter un taux de péroxydation compris entre 30 et 300 exprimé en milli-équivalents, et une teneur en gludérides oxydés comprise entre 5 et 40, la définition de cette huile en spectrophotométrie UV étant de 1 à 6 pour le facteur E 270 et de 8 à 60 pour le facteur E 232. (NF T 60 223)

Si les huiles définies ci-dessus ne comportent pas d'élément thérapeutique de quelque nature qu'il soit susceptible d'agir sur les problèmes cardiovasculaires liés à l'étiologie hémorroïdaire, celles-ci ont des propriétés antalgiques, anti-inflammatoires et cicatrisantes représentant une contribution exceptionnelle et originale d'une thérapeutique symptomatique.

L'activité anti-inflammatoire a été mise en évidence à partir d'un protocole expérimental traditionnel d'irritation locale de l'oreille du rat avec l'huile de croton ; au cours de ses expériences, l'activité de la composition objet de l'invention a été comparée à celle de l'indométacine qui est l'anti-inflammatoire utilisé classiquement ; cette étude réalisée chez le rat a permis de conclure que l'administration topique de la composition objet de l'invention était capable de limiter, de façon nette. la phlogose induite localement par un agent irritant.

L'activité antalgique de cette composition a pu être démontrée par massage de témoins avec des huiles hyperexygénées conformes à l'invention dans le cadre d'études cliniques en double aveugle, où le produit de comparaison était anesthésiant de contact clairement défini par la pharmacopée française.

Des études cliniques réalisées en double aveugle contre placebo et d'autres travaux réalisés en ouvert, ont permet de mettre en lumière une activité cicatrisante indéniable, commune à toutes les huiles d'origine végétale hyperoxygénées répondant à la définition ci-dessus.

Selon une autre caractéristique de l'invention, la ou les huile(s) hyperoxygénée(s) est(sont) choisie(s) dans le groupe formé par l'huile d'arachide, l'huile d'amande douce et l'huile de carthame.

Il a été trouvé que ces huiles présentent à un degré élevé, la triple activité anti-inflammatoire, antalgique et cicatrisante susmentionneé, et, en même temps, se distinguent par une excellente tolérance et une absence totale de toxicité.

En effet, tous les travaux effectués dans ce sens (détermination de l'indice d'irritation primaire cutanée chez le lapin, détermination de l'indice d'irritation occulaire, étude de la toxicité transmuqueuse chez le lapin, recherche d'éventuels pouvoirs allergènes...) ont amené sans exception à conclure par un constat d'absence totale d'irritation cutanée ou de toute autre tolérance allergique ou non, qu'il s'agisse de la peau ou des muqueuses.

En pharmacologie humaine, tous les travaux cliniques entrepris ont confirmé sans exception les facteurs de tolérance mis en évidence par les travaux réalisés sur l'animal.

Cette excellente tolérance est particulièrement importante dans le cas d'une compositions destinée au traitement des hémorroïdes, c'est-à-dire d'une affection dans laquelle la muqueuse est profondément atteinte et sensible à toute agression.

Parmi les huiles hyperoxydées mentionnés ci-dessus, on a trouvé que l'huile d'amande douce possédait des propriétés antalgiques et anti-inflammatoires en faisant un produit particulièrement actif dans le traitement des hémorroïdes.

L'oxygène présent dans la composition conforme à l'invention et libéré dans le derme, qui est constitué uniquement d'oxygène non toxique (c'est-à-dire dépourvu de radicaux libres) pourrait agir au niveau des terminaisons nerveuses dermiques en empêchant le processus douloureux de se former, celui-ci semblant résulter de l'anoxie au niveau des récepteurs épidermiques de la douleur.

Bien entendu, la composition médicamenteuse objet de l'invention peut n'être constituée que par une ou plusieurs huiles naturelles d'origine végétale hyperoxygénées répondant à la définition mentionnée ci-dessus ; cependant, et selon une autre caractéristique de l'invention, cette composition se présente aussi sous la forme de gels et suppositoires.

Ainsi, parmi les formes galéniques possibles de la composition conforme à l'invention, on peut noter un gel contenant entre 90 et 95 % d'huile d'amande douce hyperoxygénée et entre 4 et 8 % d'un excipient à base de silice tel que l'AEROSIL 300 commercialisé par la Société DEGUSSA FRANCE à Neuilly Sur Seine.

On a, par exemple, obtenu des résultats particulièrement satisfaisants en utilisant un gel ayant la composition suivante :
- Huile d'amande douce hyperoxygénée ..... 93,765 %
- AEROSIL 300 (marque déposée) ........... 6,235 %.

Il est remarquable de constater que les huiles hyperoxygénées contenues dans la composition objet de l'invention sont dénuées de présence de radicaux libres cytotoxiques, qui sont généralement associés à tout corps oxydé ; cette absence est de nature à justifier l'emploi de ces compositions en médecine humaine ; en effet, l'agressivité des radicaux libres sur la peau est bien connue et leur contribution à l'apparition de cancer a été fréquemment mentionnée.

Il est, en outre, essentiel de mentionner que les compositions objet de l'invention ne sont pas limitées à la présence des huiles susmentionnées et que toutes les huiles végétales péroxydées répondant à la définition ci-dessus peuvent parfaitement être utilisées dans le cadre des compositions conformes à l'invention.

L'activité des compositions conformes à l'invention sera mise en lumière grâce à l'exemple ci-dessous qui est un compte-renu d'essai en double aveugle de l'huile d'amande douce hyperoxygénée en proctologie, par comparaison à un placebo.

Tous les patients inclus dans l'essai souffraient d'une affection protologique ; la répartition est la suivante :

| | HUILE D'AMANDE DOUCE HYPER-OXYGENEE | PLACEBO |
|---|---|---|
| Anite hémorroïdaire | 5 cas | 4 cas |
| Crise hémorroïdaire aiguë | 2 cas | 5 cas |
| Hémorroïde chronique avec congestion anale persistante | 6 cas | 4 cas |
| Oedème ano-cutané spontané ou post-opératoire | 3 cas | 0 cas |
| Papillitte -- Crypto papillitte | 2 cas | 3 cas |
| Fissure anale simple ou compliquée | 3 cas | 7 cas |
| Prurit anal | 1 cas | 0 cas |
| TOTAL | 22 cas | 23 cas |

Le produit a été prescrit à raison de 10 gouttes quatre fois par jour, dont l'absorption complète par la peau était obtenue par de légers massages.

La durée du traitement a été de dix jours environ.

En fonction de l'évolution de la symptomatologie et des signes physiques on a obtenu les résultats globaux suivants :

| | HUILE D'AMANDE DOUCE HYPER- OXYGENEE | PLACEBO |
|---|---|---|
| Résultats excellents | 6 | 1 |
| Résultats bons | 12 | 4 |
| Résultats insuffisants | 4 | 11 |
| Résultats nuls | 0 | 7 |

82 % — 22 %

Ainsi, on a obtenu 18 résultats très satisfaisants ches les 22 sujets traités par l'huile d'amande douce hyperoxygénée contre 5 résultats très satisfaisants ches les 23 sujets traités par l'huile Placebo.

La tolérance clinique a, en outre, était excellente dans le groupe des sujets traités par la composition conforme à l'invention.

L'action antalgique et anti-inflammatoire du produit a été remarquable pour les sujets traités par la composition conforme à l'invention.

En conclusion, conformément à l'invention, il s'est avéré que le traitement d'hemorroïdes par l'huile hyperoxygénée sous sa forme de gel est nettement supérieur aux autres produits d'automédication.

Pour retrouver une action anti-inflammatoire ou antalgique telle qu'elle a été démontrée par les travaux cliniques pour les huiles hyperoxygénées, il faut avoir recours à des produits toxiques délivrés sur prescription médicale, puisqu'il s'agit de préparations à base de cortisone ou d'anesthésiants chimiques de contact.

**Revendications**

1°) Composition médicamenteuse destinée à être utilisée pour le traitement des hémorroïdes, caractérisée en ce qu'elle contient au moins une huile naturelle d'origine végétale hyperoxygénée par saturation en oxygène et exposition intensive et contrôlée aux ultraviolets de façon à présenter un taux de péroxydation compris entre 30 et 300 exprimé en milli-équivalents et une teneur en glycérides oxydés comprise entre 5 et 40, la définition de cette huile en spectrophotométrie UV étant de 1 à 6 pour le facteur E 270 et de 8 à 60 pour le facteur E 232.

2°) Composition médicamenteuse selon le revendication 1, caractérisée en ce que la ou les huile(s) hyperoxygénée(s) est(sont) choisie(s) dans le groupe formé par l'huile d'arachide, l'huile d'amande douce et l'huile de carthame.

3°) Composition médicamenteuse selon l'une quelconque des revendications 1 et 2, caractérisée en ce que l'huile hyperoxygénée est l'huile d'amande douce.

4°) Composition médicamenteuse selon l'une quelconque des revendications 1 à 3, caractérisée en ce qu'elle se présente sous la forme d'un gel ou d'un suppositoire.

5°) Composition médicamenteuse selon la revendication 4, se présentant sous la forme d'un gel, caractérisée en ce qu'elle contient entre 90 et 95 % d'huile d'amande douce hyperoxygénée et entre 4 et 8 % d'un excipient à base de silice.